# EUROPÄISCHE PATENTANMELDUNG

(11) **EP 1 679 117 A2**
(43) Veröffentlichungstag der Anmeldung: **12.07.2006**
(21) Anmeldenummer: 05027611.2
(22) Anmeldetag: 16.12.2005
(51) Int. Cl.: B01J 20/26, B01J 20/28, A61M 1/36, B01D 15/00

(54) **Adsorptionssystem zur Entfernung von Viren und viralen Bestandteilen aus Flüssigkeiten, insbesondere aus Blut und Blutplasma**

(30) Priorität: 10.01.2005 DE 102005001162
(71) Anmelder: HaemoSys GmbH, 07745 Jena (DE)
(72) Erfinder: Nowak, Götz, 99097 Erfurt (DE); Bucha, Elke, 99094 Erfurt (DE); Lange, Ute, 07768 Kahla (DE); Zeeb, Sven, 99441 Magdala (DE); Schumann, Alrun, 07743 Jena (DE)
(74) Vertreter: Vossius & Partner

(57) **Zusammenfassung**

Die vorliegende Erfindung betrifft ein Adsorptionssystem zur Entfernung von Viren und Virusbestandteilen, insbesondere Hepatitis C Viren, aus physiologischen Flüssigkeiten, insbesondere Vollblut oder Blutplasma, mittels extrakorporaler Adsorptionsverfahren, sowie ein Adsorbermaterial zur Verwendung im erfindungsgemäßen Adsorptionssystem.

## Beschreibung

Die vorliegende Erfindung betrifft ein Adsorptionssystem zur Entfernung von Viren und Virusbestandteilen, insbesondere Hepatitis C Viren, aus physiologischen Flüssigkeiten, insbesondere Vollblut oder Blutplasma, mittels extrakorporaler Adsorptionsverfahren, sowie ein Adsorbermaterial, umfassend poröse Polymerpartikel, zur Verwendung im erfindungsgemäßen Adsorptionssystem.

Bekanntermaßen stellen virale Infektionskrankheiten eine bedeutende Gesundheitsbedrohung dar. In vielen Fällen sind die derzeitigen prophylaktischen und therapeutischen Möglichkeiten auf diesem Gebiet noch vollkommen unbefriedigend.

Allein an Hepatitis C sind weltweit mehr als 200 Millionen Menschen erkrankt. Für diese und andere wesentliche Infektionskrankheiten, wie z.B. das Acquired Immunodeficiency Syndrome (AIDS), besteht zur Zeit keine Möglichkeit eines prophylaktischen Impfschutzes.

Die derzeitig als Standardtherapie gegen Hepatitis C eingesetzte Kombinationsbehandlung mit PEG-Interferon und Ribavirin führt nur bei 50 - 60 % der Behandelten zur Heilung, ist langwierig und kostenaufwendig. Aufgrund ihres großen Nebenwirkungspotentials führt sie bei vielen Patienten zum Therapieabbruch oder ist primär bereits kontraindiziert.

Entsprechend der weltweiten gesundheitspolitischen Bedeutung viraler Erkrankungen besteht ein großes wissenschaftliches und kommerzielles Interesse an der Entwicklung neuer antiviraler Therapien. Dieses Interesse ist nicht auf Behandlungsmethoden beschränkt, die die Therapie von Viruserkrankungen am Patienten ermöglichen, sondern erstreckt sich auch auf Verfahren, mit Hilfe derer die Übertragung von Viruserkrankungen mittels Blut- oder Plasmaspenden verhindert werden kann.

Neue Arzneimittel gegen Hepatitis C, wie z.B. Nukleosid Analoge, Hemmstoffe virusspezifischer Enzyme oder Modulatoren der Immunreaktion, befinden sich noch in frühen Phasen der klinischen Erprobung (Dev. A. et al.: Infect. Med. 21:28-36, 2004). Therapielimitierend wirkt sich vor allem das rasche Auftreten von Resistenzentwicklungen aus, das durch die hohe Mutationsrate im Virusgenom bedingt ist. Außerdem sind zur Zeit keine gesicherten Aussagen über Effektivität und Sicherheit der neuen Therapieansätze möglich. Für die bei Hepatitis C erforderliche Langzeittherapie besteht vor allem die Gefahr des Auftretens schwerer Nebenwirkungen, wie Kanzerogenität und Organtoxizität.

Adsorptive extrakorporale Behandlungsverfahren werden zur Zeit z.B. im Rahmen der Therapie schwerer Formen der Hypercholesterinämie sowie schwerer Autoimmunerkrankungen, wie der Rheumatoidarthritis und der idiopathischen thrombozytopenischen Purpura eingesetzt. Die Adsorption von Lipoproteinen, Immunglobulinen oder Immunkomplexen erfolgt hierbei bevorzugt über oberflächengebundene Liganden wie Protein A, Antikörper oder negative Ladungen der Adsorberoberfläche. Die meisten dieser Verfahren können aufgrund der schlechten Zellverträglichkeit der Adsorbermaterialien nur im Blutplasma und nicht im Vollblut durchgeführt werden.

Es gibt zahlreiche Ansätze die Anwendungsmöglichkeiten für extrakorporale Therapieverfahren zu erweitern. Im Vordergrund stehen hierbei Entwicklungen zur Entfernung weiterer pathogener Makromoleküle, z.B. aus dem Bereich der Toxine, Antikörper, Immunkomplexe oder Cytokine. Derartige Verfahren wurden auch als Möglichkeit zur Entfernung von Krankheitserregern aus Flüssigkeiten erkannt. Zur praktischen Anwendung kommen in diesem Zusammenhang bislang nichttherapeutische Verfahren, die Krankheitserreger in vitro aus Blut- oder Plasmaspenden entfernen. Bevorzugt werden hierzu Filtrationsverfahren eingesetzt. So offenbaren z.B. EP 002 949 und EP 0 517 501 den Einsatz speziell strukturierter poröser Polymermembranen zur Filtration von Viren aus verschiedenen proteinhaltigen Flüssigkeiten. EP 1 444 996 beschreibt einen Virenfilter, der die gleichzeitige Entfernung von Viren und Leukozyten ermöglicht und dessen Wirkung sich neben der Filtration auf einer Aktivierung des Komplementsystems in den Blutproben, ausgelöst durch die Membranoberfläche, bedient. Für einen in vitro Einsatz wurden auch Zellrezeptoren für Viren in einer Weise stabilisiert, die ihre Oberflächenfixation als Virusadsorbens für kontaminierte Flüssigkeiten ermöglicht (WO89/01813). Diese Verfahren sind nicht für einen direkten Einsatz am Patienten vorgesehen.

Im Gegensatz zu den in vitro Verfahren zur Dekontamination von Flüssigkeiten, Blutplasma oder Blut, bei denen Filtrationsverfahren im Vordergrund stehen, wird die Nutzbarmachung extrakorporaler Verfahren für einen therapeutischen Einsatz gegen Virusinfektionen vor allem auf der Basis adsorptiver Techniken angestrebt. Bevorzugt wird hier auf spezifische Interaktionen mit speziellen Erkennungsstrukturen auf der Virusoberfläche gesetzt, die allerdings das Problem des Auftretens von Resistenzerscheinungen in sich tragen. So wird z.B. die Entfernung von Viren durch Adsorption an oberflächengebundene monoklonale oder polyklonale Antikörper (DE 101 18 115, US 6,528,057) oder Teilen von Antikörpern (CN 1457899), die spezifisch gegen Strukturen auf der Virusoberfläche gerichtet sind, vorgeschlagen. Die Vermittlung der Adsorption über die Affinität bestimmter Lektine zu Proteinen der Virushülle (GP120 des human immunodeficiency virus (HIV)), wird in WO2004/064608 offenbart. Ebenfalls beschrieben wurde die Bindung von HIV an einen oberflächenfixierten C1-Esterase-Hemmstoff (EP 0 966 976) oder gebundene CD4-Zellrezeptoren für HIV (US 6,174,299):
Unspezifische Verfahren zur Virenadsorption verwenden chemisch modifizierte Polymeroberflächen z.B. Sulfatgruppen (EP 0 679 436) oder leicht saure oder basische Gruppen (US 5,041,079), wie sie beispielsweise in lonentauscherharzen vorliegen. In diesen Fällen kommt es bei Kontakt mit Vollblut jedoch leicht zu störenden Wechselwirkungen mit Blut- oder Plasmabestandteilen. Dies schränkt den dauerhaften Einsatz derartiger Adsorbermaterialien ein, da ihre Oberflächenchemie Aktivierungsvorgänge, insbesondere die Blutgerinnung, fördert, wodurch die Adsorbervorrichtung schon nach kurzzeitigem Einsatz undurchlässig wird. Entsprechend muss ein direkter Kontakt des Adsorbermaterials mit den zellulären Bestandteilen des Vollblutes z.B. mit Hilfe einer vorgeschalteten Filtration zu Plasma vermieden werden, wobei Blutbestandteile wie Erythrozyten, Leukozyten und Thrombozyten abgetrennt werden, und das Plasma zurückbleibt. Ebenfalls nicht für einen therapeutischen Einsatz im Vollblut geeignet ist das in WO01/40448 offenbarte Verfahren zur Viruselimination mittels Filtration durch Membranen, an deren Oberfläche sich Polymerseitenketten in bestimmter Dichte und Länge sowie mit verschiedenen funktionellen Gruppen befinden.

Eine unspezifische Verminderung der Viruslast im Blut von Hepatitis-C-Viren (HCV)-Infizierten wird während einiger, im Rahmen anderer Indikationen angewendeter extrakorporaler Verfahren beschrieben. So tritt eine Senkung der Viruslast während eines Zellapherese-Verfahrens auf, das zur Therapie der Colitis ulcerosa eingesetzt wird und Granulozyten und Monozyten durch Adsorption an raue Cellulosepartikel entfernt. Während Sawada et al. in Therapeutic Apheresis 7: 547-553, 2003 nur eine mäßige zusätzliche Verminderung der HCV-RNA beschreiben, offenbaren sie in US 6,713,252 einen therapeutischen Effekt bei Erhöhung der Behandlungshäufigkeit.

Bei Hepatitis-C-Patienten, die regelmäßig mit einer künstlichen Niere behandelt werden, wurde durchschnittlich eine geringere Viruslast als bei nichtdialysepflichtigen Patienten mit Hepatitis C beobachtet. Eine direkte Entfernung von Viren durch die Membran bestimmter Dialysatoren wird allerdings kontrovers diskutiert (Fabrizi Fet et al.: J. Nephrol.16: 467-475, 2003). Die Datenlage hinsichtlich einer möglichen Verminderung der Viruslast im Blut von HCV-Infizierten im Zusammenhang mit Lipidaphereseverfahren ist ebenfalls widersprüchlich (Marson, P. et al.: Int. J. Artif. Organs 22: 640-644, 1999; Schettler V. et al.: Eur. J. Clin. Invest. 31: 154-155, 2001). Für alle diese Verfahren wurden keine therapeutischen Effekte auf die vorbestehenden Virusinfektionen nachgewiesen.

Der aktuelle Stand der Technik begründet den Bedarf an einem effizienten, einfachen, verträglichen und ökonomisch machbaren Verfahren zur spezifischen Entfernung von Viren aus Blut oder Blutplasma von virusinfizierten Patienten, bei dem es zu keiner Resistenzentwicklung kommt und die Virusinfektion im Zusammenspiel mit körpereigenen Abwehrfunktionen zur Heilung geführt werden kann.

Der vorliegenden Erfindung liegt deshalb die Aufgabe zugrunde, eine alternative Möglichkeit zur Bekämpfung und Prophylaxe von Virusinfektionen zu entwickeln, die die Nachteile vor allem hinsichtlich Effektivität, Resistenzentwicklung und Toxizität bisheriger antiviraler Behandlungsansätze bei möglichst geringen Kosten umgeht. Die Therapieoption soll auf Virusinfektionen andwendbar sein, die mit einer längerzeitigen Virämie einhergehen. Das besondere Ziel der vorliegenden Erfindung war die Bereitstellung eines Adsorptionssystems unter Verwendung eines, blutverträglichen Adsorbermaterials, das hinsichtlich der Spezifität des Lösungsansatzes dazu geeignet ist, gezielt Viren und virale Bestandteile zu entfernen, dessen Wirkprinzip aber nicht nur auf eine oder wenige virale Oberflächenstrukturen ausgerichtet ist.

Die Aufgabe wird erfindungsgemäß gelöst durch ein Adsorptionssystem zur Entfernung von Viren und viralen Bestandteilen aus physiologischen Flüssigkeiten, insbesondere Blut oder Blutplasma, das ein partikuläres Adsorbermaterial enthält. Das Adsorbermaterial umfasst Partikel mit einem Durchmesser von 20 bis 500 µm, die eine Polymermatrix mit Durchgangsporen aufweisen.

Als besonders geeignet für die Entfernung von Viren erwiesen sich, in Abhängigkeit von der Virenspezies, Porenradien der Durchgangsporen zwischen 25 und 1000 nm.

Die Adsorption vollzieht sich während des Kontaktes der Flüssigkeit, wie virushaltigem Vollblut oder Blutplasma, mit dem Adsorbermaterial, das beispielsweise als Füllung einer Kartusche oder eines kartuscheähnlichen Behältnisses bereitgestellt werden kann. Die unter Anwendung des erfmdungsgemäßen Adsorptionssystems gesenkte Viruslast dient der Therapie und der Prophylaxe von Virusinfektionen.

Um eine Verminderung der Virenzahl im Organismus infizierter Patienten zu erreichen, wird das Adsorptionssystem vorzugsweise im Rahmen eines extrakorporalen Verfahrens mit dem Blut oder Blutplasma des Patienten in Kontakt gebracht. Beispielsweise kann es mittels eines extrakorporalen Kreislaufes in die Blut- oder Plasmazirkulation geschaltet werden. Das erfindungsgemäße System eignet sich jedoch gleichermaßen zur Behandlung von physiologischen Flüssigkeiten, die nicht oder nicht unmittelbar in den Organismus des Patienten zurückgeführt werden, wie z.B. Blut- oder Plasmaspenden.

Überraschenderweise wurde festgestellt, dass Polymerpartikel mit speziellen porösen Eigenschaften in der Lage sind, Viren in einer Weise zu adsorbieren, die deren irreversible Entfernung aus einer wässrigen Lösung, aus Blutplasma oder Blut ermöglicht. Der Einsatz poröser Partikel zur Entfernung verschiedener pathologischer Substanzen oder Toxine im Bereich kleinerer und mittlerer Molekülgrößen bis ca. 30.000 Dalton aus Blutplasma oder Blut mittels extrakorporaler therapeutischer Verfahren wird in EP 1 115 456 offenbart. Hinweise darauf, dass mit Hilfe poröser, partikulärer Adsorptionsmaterialien derartig komplexe biologische Strukturen wie Viren ohne Einsatz biologischer Erkennungsstrukturen, wie etwa oberflächengebundener Antigene oder Antikörper, effizient selbst aus Vollblut entfernt werden können, gab es bislang jedoch nicht.

Bei den Partikeln des Adsorbermaterials handelt es sich um Partikel mit einer Polymermatrix, die von Durchgangsporen durchsetzt sind. Derartige Durchgangsporen durchziehen die Polymermatrix in Form von Kanälen, die im Inneren der Partikel getrennt verlaufen, sich verzweigen und/oder oder sich kreuzen können. Sich verzweigende und/oder kreuzende Kanäle sind im Hinblick auf das Strömungsverhalten der zu behandelnden Flüssigkeit bevorzugt. Die Durchgangsporen sind so zur Partikeloberfläche hin geöffnet, dass eine Durchströmung der Partikel gewährleistet ist. Bei den Partikeln der vorliegenden Erfindung sollte es sich jedoch nicht um Mikrokapseln mit einer porösen Oberfläche handeln, d.h. Partikel, bei denen eine relativ dünne Schale (bis ca. 5 % des Gesamtdurchmessers) einen hohlen Kern umschließt, der kein Matrixmaterial enthält.

Der Begriff "Porenradius", wie er hier verwendet wird, bezeichnet eine über alle Poren und deren Länge gemittelte Porenweite von zylinderförmigen Kanälen, wie er modellhaft mit einer standardisierten Messmethode (inverse Größenausschlusschromatographie) bestimmt werden kann. Dementsprechend liegt der Porenradius in Abhängigkeit vom zu entfernenden Material bevorzugt zwischen 25 und 1000 nm, besonders bevorzugt zwischen 50 und 600 nm. Für die meisten Viren findet ein Radius von 80 bis 450 nm Anwendung.

Es wurde festgestellt, dass für eine effektive Entfernung von Viren der Porenradius bevorzugt größer ist als der Radius des Virus. Für den Hepatitis C Virus mit einem Durchmesser von 40 bis 75 nm ergibt sich ein bevorzugter Porenradius von 80 bis 450 nm.

Die Porosität der Partikel (ausgedrückt als das Verhältnis des Porenvolumens zum Gesamtvolumen der Matrix und der darin enthaltenen Poren) liegt im Hinblick auf eine effiziente Abtrennung von Viren oder Virusbestandteilen vorzugsweise zwischen 0,25 und 0,75, bevorzugt zwischen 0,4 und 0,6.

Das erfindungsgemäße Adsorptionssystem umfasst Partikel mit einem mittleren Durchmesser von 20 µm oder mehr, bevorzugt 50 µm oder mehr, z.B. 80 µm oder mehr oder 100 µm oder mehr. Die Obergrenze des mittleren Durchmessers liegt bei 500 µm, bevorzugt bei 350 oder 250 µm. Besonders bevorzugt sind Partikel mit einem mittleren Durchmesser von 100 bis 250 µm. Die Partikel weisen vorteilhafterweise eine sphärische Gestalt auf.

Durch die bevorzugte Partikelgröße ist gewährleistet, dass in dem erfindungsgemäßen Adsorptionssystem zwischen den Partikeln des Adsorptionsmaterials Zwischenräume ausreichender Größe vorhanden sind. Dies ist z.B. bei der Behandlung von Vollblut oder anderer heterogener Flüssigkeiten von Bedeutung, da größer dimensionierte Bestandteile des Blutes wie Erythrozyten, Leukozyten und Blutplättchen diese Zwischenräume benötigen, um das Adsorptionssystem zu passieren, während das Plasma zusammen mit den Viren bevorzugt durch die Durchgangsporen der Partikel fließt. Bevorzugt sollten diese Zwischenräume durch eine geeignete Auswahl der Partikelgröße gerade so eingestellt sein, dass die vorgenannten zellulären Blutbestandteile hindurchtreten können und dass bei diesem Durchtritt ein Strömungswiderstand entsteht, der einen innerporigen Transfer des virushaltigen Plasmas gewährleistet.

Die spezifische Oberfläche der Partikel des Adsorptionsmaterials liegt in der Regel bei 50 bis 1000 m²/g, bevorzugt bei 50 bis 500 m²/g, besonders bevorzugt bei 50 bis 300 m²/g oder 50 bis 150 m²/g.

Die in dem Adsorptionssystem vorhandenen porösen Partikel weisen eine Polymermatrix auf, wobei von diesem Begriff auch Copolymere aus zwei oder mehreren unterschiedlichen Monomeren umfasst werden. Hinsichtlich geeigneter Polymere sind die erfindungsgemäß eingesetzten Partikel nicht eingeschränkt, sofern das Polymermaterial bei Kontakt mit physiologischen Flüssigkeiten keine unverträglichen Produkte abscheidet oder selbst mit der betreffenden Flüssigkeit unverträglich ist und sich zur Bereitstellung einer porösen Matrix eignet. Um eine unspezifische Anbindung z.B. von Proteinbestandteilen, wie Fibrinogen, oder von zellulären Bestandteilen, wie Thrombozyten, auf der Partikeloberfläche zu erschweren, besitzen die in der Matrix verwendeten Polymere bevorzugt keine Gruppen, die unter physiologischen Bedingungen in ionischer Form vorliegen oder ionisiert werden können.

Die für die poröse Polymermatrix der Partikel des Adsorptionsmaterials verwendeten Materialien sollten bevorzugt eine ausreichende Rigidität aufweisen, damit keine Verformung der Partikel durch den Druck der durchströmenden Flüssigkeit auftritt. Die Polymermatrix muss in wässrigen Lösungen dimensionsstabil sein, d.h. die Partikel- bzw. Porengröße sollte sich nicht durch Aufquellen der Partikel ändern.

Beispiele für geeignete Polymere zur Bereitstellung der porösen Polymermatrix der Partikel des Adsorptionsmaterials sind Vinylverbindungen, bevorzugt Methacrylsäureester oder Styrole, bzw. deren Copolymere mit geeigneten Vernetzungsmitteln, wie Ethylenglycoldimethacrylat oder Divinylbenzol.

Im Hinblick auf eine gegebenenfalls durchzuführende weitergehende Behandlung der porösen Polymermatrix mit einem Agens, das der Partikeloberfläche zusätzliche Hämokompatibilität verleiht, kommen in der porösen Polymermatrix bevorzugt Polymere zum Einsatz, wie sie auch in der EP 0 975 680 oder der EP 1 282 695 beschrieben sind. Zu deren Herstellung wird mindestens ein Monomertyp verwendet, der neben einer polymerisierbaren Doppelbindung oder einer polykondensierbaren funktionellen Gruppe eine weitere Carbonylgruppe in Form eines Ketons oder eines Carbonsäurederivats enthält, die nicht an der Polymerisationsreaktion teilnimmt. Bevorzugt enthält das Polymer Strukturelemente der Formel (A): 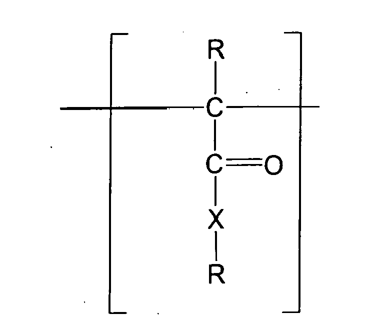 wobei die Reste R gleich oder verschieden sein können und einen Alkyl oder Arylrest oder ein Wasserstoffatom darstellen. Der Alkylrest kann linear, verzweigt oder cyclisch sein und besteht bevorzugt aus 1 bis 20 Kohlenstoffatomen, besonders bevorzugt aus 1 bis 8 oder 1 bis 4 Kohlenstoffatomen. Der Arylrest besteht bevorzugt aus 6 bis 18, besonders bevorzugt aus 6 bis 12 Kohlenstoffatomen. Der bivalente Rest X ist fakultativ und bedeutet O, NR oder CH₂, wobei R wie vorstehend definiert ist. Wie vorstehend erwähnt sind jedoch solche Vertreter der vorstehenden Struktureinheit (A) bevorzugt, die unter physiologischen Bedingungen nicht in ionischer Form vorliegen und nicht ionisiert werden können. Besonders bevorzugt als Gruppierung -X-R ist daher ein Alkoxyrest, wobei dessen Alkylteil wie vorstehend definiert ist und bevorzugt von einer Methylgruppe gebildet wird.

Besonders bevorzugte Monomere zur Bereitstellung der Strukturelemente (A) sind Alkylmethacrylate mit einem Alkylrest, der vorzugsweise 1-6 C-Atome umfasst, wie z.B. Methylmethacrylat, Ethylmethacrylat oder Propylmethacrylat. Weiterhin können Vinylacetat, Cyclohexylmethacrylat oder Phenylmethacrylat bzw. Gemische der vorgenannten Monomere eingesetzt werden. Insbesondere bevorzugt wird für die Polymermatrix der Partikel des Adsorptionsmaterials jedoch ein Polymer, das Einheiten enthält, die sich von Methylmethacrylat als Monomerem ableiten.

Auch Copolymere oder Polymermischungen aus beliebigen Anteilen der vorstehend genannten Polymere mit Strukturelementen der Formel (A) untereinander oder mit einer oder mehreren zusätzlichen Polymerkomponente(n), beispielsweise Polystyrol, Polyacrylnitril oder Polyamiden können eingesetzt werden. Bevorzugt beträgt der Anteil der Monomere, die nach der Polymerisation das Strukturelement (A) zur Verfügung stellen, an der Gesamtmenge der eingesetzten Monomere mindestens 5 mol%, zum Beispiel 10 oder 20 mol%. Besonders bevorzugt beträgt dieser Anteil mindestens 30 mol%, und ganz besonders bevorzugt mindestens 50 mol%.

Bevorzugt kommen in der porösen Polymermatrix vernetzte Polymere zum Einsatz. Bei ihrer Herstellung werden auf herkömmliche Weise zusammen mit den vorstehend genannten Monomeren Vernetzungsmittel, d.h. netzwerkbildende bi- oder multifunktionelle Monomere eingesetzt, die beispielsweise zwei oder mehr polymerisierbare Gruppen aufweisen und bei der Polymerisation zugegeben werden. Lediglich illustrative Beispiele für solche Monomere sind Divinylbenzol, Ethylenglycoldimethacrylat oder Butylenglycoldimethacrylat, zuzüglich für sie geeigneter netzwerkbildender Lösungsvermittler. Ein besonders bevorzugtes Copolymer zur Bereitstellung der porösen Partikel ist Polymethylmethacrylat-coethylenglycol-dimethacrylat. Das molare Verhältnis vom Methylmethacrylat und Ethylenglykoldimethacrylat liegt bevorzugt bei 4:2 bis 0,25:1 und besonders bevorzugt bei 2:1 bis 1:2.

Partikel mit einer Polymermatrix der erforderlichen Partikel- und Porengröße können nach bekannten Verfahren hergestellt werden. Geeignet ist beispielsweise die Suspensionspolymerisation, bei der ein erstes Monomer sowie evtl. ein Vernetzungsmittel sowie eine zweite (äußere) Phase benötigt werden, wobei Monomer und Vernetzungsmittel in der äußeren Phase nicht löslich sein dürfen. Der als Initiator verwendete Radikalstarter ist im Monomeren gelöst. Die Monomermischung wird in der äußeren Phase (z.B.Wasser) durch Einwirkung mechanischer Energie, praktischerweise Rühren, fein verteilt. Ein Zusammenfließen der Monomerphase und Entmischen wird durch Zusatz wasserlöslicher Schutzkolloide verhindert. Bei der Suspensionspolymerisation entstehen sphärische Polymerperlen, wobei die Größe der einzelnen. Teilchen durch Variation der Herstellungsbedingungen wie Rührgeschwindigkeit, Art und Menge des Schutzkolloids in weiten Bereichen eingestellt werden kann. Durch Zusatz sogenannter Porogene, welche Einfluss auf die Mischungsthermodynamik während der Polymerisation nehmen, ist es möglich, Poren gezielt in das Material einzubauen. Grundvoraussetzung ist dabei, dass das Polymere in seinem Monomeren während fortschreitender Reaktion unlöslich wird und ausfällt. Dies kann beispielsweise durch Zugabe eines Vernetzermonomers erreicht werden. Durch Zugabe eines inerten Quellungsmittel kann der Gelpunkt, und damit der Zeitpunkt des Ausfallens gezielt verschoben weren, wodurch es möglich ist, die Porengröße auf den gewünschten Wert einzustellen. Eine weitere Einflussmöglichkeit ergibt sich durch weitere Zumischung eines inerten Fällungsmittels. Die porosimetrischen Kenngrößen wurden mit der Methode der inversen Größenausschlusschromatographie bestimmt (s. hierzu Gorbunov, A et al.; J Chromatogr 448, 307 (1988)). Zur Herstellung poröser Partikel vgl. Seidl, J., Malinstky, J.; Dusek, K; Heitz, W.; Adv. Polym. Sci.; 5; 113-213 (1967); Logemann, H. et al.; in: Houben/Weyl; Methoden der Organischen Chemie; Band 14; 133-438 (1967); Polymerisation der Acryl- und Methycrylsäure, ihrer Salze, Halogenide und Anhydride; Völker, T. et al.; ibid; 1018-1072; Synthesis and characterization of porous polymers; Sherrington, D.C.; Makromol. Chem., Macromol. Symp.; 70/71, 303 (1993); Porous Poly-HEMA beads prepared by suspension polymerization in aqueous medium; Horak, D.; Ledniki, F.; Rehak, V.; Svec, F.; J. Appl. Polym. Sci.; 49, 2041 (1993); Chromatographic properties of macroporous beads from poly (GMA-co-EDMA); Horak, D.; Svec, F.; Tennikowa, T. B.; Nahunek, M.; Angew. Makromol. Chem.; 195, 139 (1992); Dispersion copolymerization of methyl methacrylate and acrylic acid in polyr media; Huang, J. X.; Yuan, X. Y.; Yu, X. L.; Zhang, H. T.; Polym. Int; 52, 819 (2003); Preparation and porosity characterization of highly cross-linked polymer resins derived from multifunctional methacrylate monomers; Rohr, T.; Knaus, S.; Grber, H.; Shernngton, D. C.; Macromolecules; 35, 97 (2002); Preparation by suspension polymerization of porous beads for enzyme immobilization; Skovby, M. H. B.; Knops, J.; J. Appl. Polym. Sci.; 39, 169 (1990); Reactive polymers XXXIII; The influence of the suspension stabilizer on the morphology of a suspension polymer; Horak, D.; Pelzbauer, Z.; Svec, F.; Kalal, J.; J. Appl. Polym. Sci.; 26, 3205 (1981);New designs of macroporous polymers and supports; from separation to biocatalysis; Svec, F.; Frechet, J. M. J.; Science; 273, 205 /1996).

Eine zusätzliche Verbesserung der Hämokompatibilität der Partikel und/oder eine Erhöhung der virusadsorbierenden Kapazität insbesondere bei Kontakt mit Blut als bevorzugt zu behandelnder Flüssigkeit kann durch ein Aufbringen von oberflächenpassivierenden Wirkstoffen, wie Inhibitoren der Blutplättchen- bzw. Zellaktivierung, auf die Partikeloberfläche erreicht werden. Bevorzugt werden solche Wirkstoffe bei ihrem Einsatz auf die äußeren und inneren Oberflächen (d.h. die Poreninnenflächen) der porösen Polymermatrix aufgebracht. Der Begriff der Hämokompatibilität und Wirkstoffe zu deren Verbesserung sind dem Fachmann geläufig, vgl. z.B. Vienken J: Biological processes related to blood-biomaterial interaction; in: Blood-Material Interaction, International Faculty For Artificial Organs (INFA), Glasgow, Krems, 1998.

Geeignete Verfahren, die aus dem Stand der Technik bekannt sind, schließen ein: Auftragen von hochmolekularem Poly(N-trifluoralkoxy)phophazen, Behandlung der porösen Partikel mit einer Phophazenlösung in einem organischen Lösungsmittel und Verdampfen des Lösungsmittels oder die elektrostatische oder kovalente Anbindung geeigneter oberflächenpassivierender Wirkstoffe, wie z.B. Heparin, an entsprechend funktionalisierte Oberflächen.

Im Rahmen der vorliegenden Erfindung werden die Hämokompatibilität verbessernde Wirkstoffe jedoch bevorzugt mit Hilfe der Interaktion zwischen speziellen Polymeren und speziellen Linkern auf die poröse Polymermatrix aufgebracht, die in EP 0 975 680 und EP 1 282 695 beschrieben ist. EP 1 282 695 offenbart dabei bereits blutkompatible Polymeroberflächen, die auch zur Bereitstellung hämokompatibler Partikel für die vorliegende Erfindung geeignet sind. Danach werden poröse Polymerpartikel, die die vorstehend beschriebenen Strukturelemente (A) und besonders bevorzugt von Alkylmethacrylaten abgeleitete Einheiten umfassen, mit Wirkstoffen in Kontakt gebracht, die die Hämokompatibilität verbessern, und die einen Linker tragen, wie er in den vorstehend genannten Veröffentlichungen beschrieben ist.

Bevorzugt werden als Linker Polyalkylenglykole, Polyalkylenimine, Polyalkylenamine oder Polyalkylensulfide, sowie Polyoxazilline eingesetzt, wobei Polyalkylenglykole besonders bevorzugt sind. Der mittlere Polymerisationsgrad dieser polymeren Linker liegt bevorzugt unterhalb von 300, besonders bevorzugt unterhalb von 150. Die Untergrenze liegt im allgemeinen bei einem Wert von 5, bevorzugt bei 10, wobei die bevorzugten Polymerisationsgrade innerhalb der vorgenannten Bereiche mit der Wahl der Linkergrundbausteine variieren können. Insbesondere bevorzugt werden als Linker Polyethylenglykole (PEG) eingesetzt. Um eine optimale Stabilität zu gewährleisten sind sie kovalent mit dem Wirkstoff verknüpft, der die Hämokompatibilität verbessert. Durch direkten Kontakt des linker-gebundenen Wirkstoffes mit einer Polymeroberfläche, die das erforderliche Strukturelement aufweist, ergibt sich eine linkervermittelte Bindung zwischen Wirkstoff und Polymer, die unter physiologischen Bedingungen hoch stabil ist.

Die vorliegende Erfindung kann die technologischen Möglichkeiten aus EP 0 975 680 und EP 1 282 695 damit bevorzugt zur Verhinderung der Interaktion der Partikeloberfläche mit Blutbestandteilen bei gleichzeitiger Erhaltung oder Erhöhung der Kapazität der Virusadsorption nutzen. Basierend auf den in EP 0 989 856 und EP 1 282 695 vorgetragenen Erkenntnissen werden hierzu Linker-gekoppelte Polyorganosiloxane und andere die Hämokompatibilität der Oberflächen verbessernde Substanzen in einer festgelegten Konzentration auf der Partikeloberfläche während eines einfachen Inkubationsvorganges fixiert. Besonders geeignet sind in diesem Zusammenhang Polyorganosiloxane, die nach chemischer Bindung an bestimmte Linker (z.B. PEG), vorzugsweise bei 20°C, in Mengen von 0,1 µg/ml bis 100 mg/ml, vorzugsweise von 1mg/ml bis 100 mg/ml oder bis 10mg/ml wasserlöslich sind.

Geeignete die Hämokompatibilität verbessernde Substanzen umfassen beispielsweise Cholesterin oder Polydimethylsiloxane, die mit einem der vorstehend genannten Linker wie z.B. PEG oder einem Mischpolymer aus PEG und Polypropylenglykol gekoppelt sind. Daneben können als hämokompatibilitätsverbessernde Substanzen auch PEG oder PEG/Polypropylenglykol Mischpolymere enthaltende Substanzen, wie PEG-Derivate von Fettsäureestern mehrwertiger Alkohole ( z.B. PEG-Glycerinfettsäureester), PEG-Sorbitanfettsäureester (z.B. Tween-Produkte), PEG-Fettsäureester, PEG-Lipide oder PEG-Proteine (z.B. PEG-Albumin) eingesetzt werden. Ebenso können speziell die Thrombogenität der Oberflächen vermindernde antikoagulative Wirkstoffe wie beispielsweise PEG-Thrombininhibitoren zur Verbesserung der Hämokompatibilität beitragen.

Zur effizienten Entfernung von Viren und Virenbestandteilen benötigen die hier vorgestellten porösen Partikel keine auf ihrer Oberfläche immobilisierten Erkennungsstrukturen. Sie sind vorzugsweise vor ihrem Kontakt mit der zu behandelnden Flüssigkeit im wesentlichen frei von Proteinen oder Nukleinsäuren, die eine Bindung von Viren oder Virenbestandteilen fördern können. Im wesentlichen frei heißt in diesem Zusammenhang, das eventuell auftretende Verunreinigungen/Restmengen solcher biologischen Moleküle nicht zur Adsorption der Viren oder Virenbestandteile beitragen. Die Partikel können auch vollständig frei sein von Proteinen oder Nukleinsäuren.

Das Adsorptionssystem der vorliegenden Erfindung umfasst vorzugsweise ein festes oder flexibles Gehäuse, durch das die zu behandelnde Flüssigkeit hindurchströmen kann und das die vorstehend beschriebenen porösen Partikel als Adsorbermaterial enthält. Die porösen Partikel im Gehäuse sollten bevorzugt nicht miteinander verbunden sein, sondern lediglich als lose Partikel vorliegen. Es sollte eine gleichmäßige Umströmung der Partikel mit durchgeleiteten Flüssigkeiten gewährleistet sein. Geometrie und Material des Gehäuses sind unter Berücksichtigung ihrer Hämokompatibilität frei wählbar. Beispielsweise kann es sich bei dem Gehäuse um eine Kartusche handeln. Sie kann beliebige Formen aufweisen und beispielsweise zylindrisch sein. Um einen kontinuierlichen Durchfluss zu gewährleisten, besitzt das Gehäuse vorzugsweise einen Einlass, der den Eintritt von Flüssigkeiten, insbesondere Körperflüssigkeiten, wie Blut oder Plasma, in das Gehäuse erlaubt und den Kontakt der Flüssigkeiten mit dem Polymerpartikeln gewährleistet, und einen Auslauf, der das Abfließen der Flüssigkeit nach dem Kontakt mit den Polymerpartikeln erlaubt. Das Adsorptionssystem besitzt weiterhin vorzugsweise eine oder mehrere Vorrichtungen, die geeignet sind, den Partikelaustritt aus dem Gehäuse zu verhindern, wie z.B. Filter oder Fritten. Sie halten die Partikel im Gehäuse, gewährleisten aber gleichzeitig einen ungehinderten Durchtritt der zu behandelnden Flüssigkeit. Das Adsorptionssystem kann so beschaffen sein, dass es in einen extrakorporalen Kreislauf eingebunden werden kann.

Das erfindungsgemäße Adsorptionssystem kann mittels eines extrakorporalen Kreislaufes in die Blut- oder Plasmazirkulation geschaltet werden. Es eignet sich jedoch auch zur Behandlung von Blut- oder Plasmaspenden oder anderer physiologischer Flüssigkeiten, die einem Organismus entnommen, jedoch nicht oder nicht unmittelbar nach dem Durchlaufen der Einheit dorthin zurückgeführt werden. Das erfindungsgemäße System kann allein oder in Verbindung mit anderen Einheiten, wie z. B. Hämodialyseeinheiten, benutzt werden. Der Flüssigkeitsstrom durch das System kann mit oder ohne Hilfe einer Pumpe gewährleistet werden.

Mit Hilfe des vorstehend beispielhaft genannten extrakorporalen Kreislaufsystems wird Blut oder Blutplasma von Patienten aus einem entsprechend kanülierten Blutgefäß durch das Adsorptionssystem geleitet. Dabei werden im Blut oder Plasma enthaltene Viren an den porösen Partikeln des Adsorbermaterials adsorbiert und damit aus der Strömung entfernt, bevor das Blut oder Plasma zurück in den Organismus geleitet wird. Das Verfahren kann in bestimmten Zeitabständen so lange wiederholt werden, bis die so erzielte Senkung der Viruslast die Infektion zur Besserung und Heilung führt. Eine Kombination mit anderen Therapiestrategien ist möglich.

Mit Hilfe des erfindungsgemäßen Adsorptionssystems werden Viren und Virenbestandteile irreversibel aus den zu behandelnden physiologischen Flüssigkeiten entfernt, d.h. die herausgefilterten Bestandteile sind unter physiologischen Bedingungen fest an das Adsorbermaterial gebunden.

Überraschenderweise lassen sich mit Hilfe des erfindungsgemäßen Adsorptionssystems Viren und Virenbestandteile mit hoher Effizienz aus diversen physiologischen Flüssigkeiten und selbst aus Blut entfernen, ohne dass es dabei einer vorherigen Abtrennung zellulärer Blutbestandteile, z.B. durch Filtration des Blutes zur Bereitstellung von Plasma, bedarf. So kann bereits nach einmaligem Durchlaufen des Adsorptionssystems der Virustiter einer Blutprobe um mehr als 90% gesenkt werden

Das Adsorptionssystem und das Adsorbermaterial der vorliegenden Erfindung eignen sich in ausgezeichneter Weise zur Entfernung von Viren und Virenbestandteilen aus physiologischen Flüssigkeiten. Als Beispiele zu nennen sind Vertreter der Familien Hepadna-, Flavi-, Coroma, Filo-, Bunuya-, Arena- und Retroviridae. Insbesondere sollen mit Hilfe der vorliegenden Erfindung neben Hepatitis C auch Erkrankungen wie z.B. Hepatitis B, AIDS oder virale haemorrhagische Fieber behandelt werden. Weitere biologische Materialien, für deren Entfernung aus physiologischen Flüssigkeiten die Adsorptionseinheit und das Adsorbermaterial der vorliegenden Erfindung angepasst werden können, sind beispielsweise RNA-Bruchstücke, Prionen, Plasmide oder andere biologische Nanopartikel.

Erfindungsgemäß können das hier beschriebene Adsorptionssystem und das hier beschriebene Adsorbermaterial zur Behandlung von Krankheiten und insbesondere von Viruserkrankungen verwendet werden, die durch die vorstehenden Erreger ausgelöst werden. Weiterhin können das Adsorptionssystem und das Adsorbermaterial zur Herstellung eines Heilmittels/Medizinproduktes zur Behandlung dieser Krankheiten verwendet werden.

Die nachfolgenden Beispiele sollen die Erfindung näher erläutern, ohne sie jedoch darauf zu beschränken.

### Herstellungsbeispiel 1:

Allgemeine Herstellungsvorschrift für sphärische, etwa 100 µm große Partikel mit Porenradien von etwa 80 nm

In einem beheizbaren Reaktorgefäß legt man eine Lösung von 0,5 g Polyvinylalkohol mit 88% Hydrolysegrad sowie 0,1 g Polyvinylalkohol mit 98% Hydrolysegrad in 400 ml Wasser vor und gibt unter Rühren eine Mischung von 10 g Methylmethacrylat, 10 g Ethylenglykoldimethacrylat, 0,5 g AIBN, 10 ml Cyclohexanol, 20 ml Octanol zu. Diese Mischung wird dann bei 70°C unter Rühren polymerisiert, bis vollständiger Umsatz erreicht ist. Die Partikel werden mehrfach mit Wasser und Methanol gewaschen, in einem Soxhlet-Extraktor mit Methanol und Aceton behandelt und bei 100°C im Vakuum bis zur Gewichtskonstanz getrocknet.

### Herstellungsbeispiel 2:

Allgemeine Herstellungsvorschrift für sphärische, etwa 100 µm große Partikel mit Porenradien von etwa 450 nm

In einem beheizbaren Reaktorgefäß legt man eine Lösung von 0,75 g Polyvinylalkohol mit 88% Hydrolysegrad sowie 10 g Natriumsulfat in 800 ml Wasser vor und gibt unter Rühren eine Lösung von 10 g Methylmethacrylat, 10 g Ethylenglykoldimethacrylat, 0,5 g AIBN, 18,3 ml Butylacetat, 36,6 ml Octanol zu. Danach werden 10 g Calciumphosphat zugegeben und die Mischung unter Rühren bei 70°C polymerisiert, bis vollständiger Umsatz erreicht ist.

Die Partikel werden mit Wasser, Salzsäure sowie mehrfach mit Wasser und Methanol gewaschen, in einem Soxhlet-Extraktor mit Methanol und Aceton behandelt und bei 100°C im Vakuum bis zur Gewichtskonstanz getrocknet.

### Herstellungsbeispiel 3:

Allgemeine Herstellungsvorschrift für sphärische, etwa 100 µm große Partikel mit Porenradien von etwa 130 nm.

In einem beheizbaren Reaktorgefäß legt man eine Lösung von 1 g Polyvinylalkohol mit 88% Hydrolysegrad in 800 ml Wasser vor und gibt unter Rühren eine Mischung von 10 g Methylmethacrylat, 10 g Ethylenglykoldimethacrylat, 0,5 g AIBN, 18,3 ml Toluol, 36,6 ml Octanol zu. Diese Mischung wird dann bei 70°C unter Rühren polymerisiert, bis vollständiger Umsatz erreicht ist. Die Partikel werden mehrfach mit Wasser und Methanol gewaschen, in einem Soxhlet-Extraktor mit Methanol und Aceton behandelt und bei 100°C im Vakuum bis zur Gewichtskonstanz getrocknet.

### Herstellungsbeispiel 4

Zur Veränderung der Partikelgröße werden die Beispiele 1 bis 3 wiederholt, wobei als Schutzkolloide in der wässrigen Phase Polyvinylalkohole mit Molmassen von 30.000 bis 200.000 und Hydrolysegraden von 70% bis 98% sowie Polyvinylpyrrolidon mit Molmassen von 10.000 bis 100.000, Polyacrylamid, Hydroxyethylcellulose, Polyacrylsäure-Natrium verwendet werden.

### Herstellungsbeispiel 5

Zur Veränderung der Partikelgröße werden die Herstellungsbeispiele 1 bis 3 wiederholt, wobei die Menge der wässrigen Phase zwischen 200 und 800 mL sowie das Verhältnis von wässeriger zu organischer Phase von 20:1 bis 5:1 variiert wird.

### Herstellungsbeispiel 6

Zur Einstellung der Porengröße werden die Herstellungsbeispiele 1 bis 3 wiederholt, wobei das Verhältnis von Monomer zu Porogen von 2: bis 1:5 verändert wird.

### Herstellungsbeispiel 7

Zur Änderung der Porengröße und Beeinflussung der Porenmorphologie werden die Herstellungsbeispiele 1 bis 6 wiederholt, wobei als porogene Komponente in der organischen Phase außer Cyclohexanol und Butylacetat auch Toluol, Cyclohexanon, Butanon, Xylol, Trichlormethan, Ethylacetat, lineares Polymethylmethacrylat, oder auch ein Gemisch aus mehreren dieser Komponenten verwendet wird.

### Herstellungsbeispiel 8

Zur Änderung der Porengröße und Beeinflussung der Porenmorphologie werden die Herstellungsbeispiele 1 bis 6 wiederholt, wobei als porogene Komponente in der organischen Phase außer Octanol auch andere primäre Alkohole sowie Alkane mit sechs und mehr Kohlenstoffatomen oder auch Mischungen dieser Komponenten verwendet werden.

### Herstellungsbeispiel 9

Zur Verbesserung der Partikelqualität und Beeinflussung der Größenverteilung werden die Herstellungsbeispiele 4 bis 6 wiederholt, wobei Menge und Art des der Wasserphase zugesetzten Salzes verändert wird. Außer Natriumsulfat kommt auch Natriumchlorid, Calciumchlorid, Kupferchlorid, Dinatriumhydrogenphosphat, Natriumacetat sowie außer Calciumphosphat auch Magnesiumcarbonat, Calciumcarbonat und hochdisperse Kieselsäure zum Einsatz. Die Menge beträgt bis 20g.

### Beispiel 10

Die Beispiele 1 bis 6 werden wiederholt, wobei als Monomere außer Methylmethacrylat auch Ethylmethacrylat, Butylmethacrylat, 2-Ethylhexylmethacrylat, Cyclohexylmethacrylat, Phenylmethacrylat, Styrol, 2-Hydroxyethylmethacrylat, Methacrylsäureamid, 2-Vinylpyrrolidon sowie außer Ethylenglykoldimethacrylat auch Divinylbenzol, Butylenglykoldimethacrylat sowie Gemische der genannten Komponenten verwendet werden. Auf diese Weise kann die Oberflächencharakteristik verändert werden.

Die Wirksamkeit der erfindungsgemäßen Adsorptionseinheit gegen verschiedene Virenspezies wurde beispielhaft für das Bovine Virusdiarrhoe Virus (BVDV) und das Hepatitis C Virus (HCV) nachgewiesen.

### Beispiel 11. Virustiterbestimmung

Die Virustiter des Bovinen Virusdiarrhoe Virus (BVDV) wurden durch Endpunktverdünnungsmethode zur Infektiositätsmessung (Virustitration) bestimmt. Die Kalkulation des Titers als logKID₅₀/ml (KID₅₀: Kultur-infektiöse Dosis zur Basis 50%) erfolgte nach Kaerber (Arch. Exp. Pathol. Pharmakol. 1931: 162, 480).

Die Virustiter von Hepatitis C Virus (HCV) wurden mit Hilfe des quantitativen HCV-Nachweises (Einheit: IU/ml) durch die Methode der Signalamplifikation mittels b-DNA-Verfahren (branched-DNA-Verfahren) bestimmt.

### Beispiel 12 Abreicherung von BVDV aus einer Virussuspension

300 mg Polymerpartikel (hergestellt analog Herstellungsbeispiel 1) mit einem mittleren Porenradius von 81 nm, einer Porosität von 0,45 sowie einer spezifischen Oberfläche von 608 m²/g wurden in PBS (phosphate buffered saline) suspendiert. Die Suspension wurde in eine Säule aus Kunststoffmaterial (Durchmesser 1 cm, Höhe des Partikelbettes 2,2 cm, Volumen des Partikelbettes 1,7 ml) gefüllt. Zur Vermeidung des Austretens von Partikeln wird das Partikelbett am oberen und unteren Ende durch Filtergewebe mit einer Maschenweite von 40 µm begrenzt. Das Totvolumen der mit Partikeln gefüllten Säule einschließlich der Anschluß- und Ablaufschläuche beträgt 2,5 ml. Die Partikel wurden mit PBS konditioniert.

Mit Hilfe einer Spritzenpumpe wurde 1 ml Virussuspension in PBS mit einem Virustiter (angegeben als logKID₅₀/ml) von 5,8 mit einem konstanten Fluß von 0,25 ml/min auf die Partikel-gefüllte Säule gepumpt. Anschließend wurde die Säule mit 5 ml PBS (0,25 ml/min) eluiert. Das Eluat (6 ml) wurde aufgefangen. Danach wurde die Säule mit 6 ml PBS (0,25 ml/min) gewaschen, die Waschlösung wurde ebenfalls aufgefangen. Waschlösung und Eluat wurden bis zur Bestimmung des Virustiters bei -80°C eingefroren. Als Vergleichsprobe wurde eine 1:6 (entsprechend der Verdünnung der Virussuspension nach Elution von der Säule) mit PBS verdünnte Virussuspension mitgeführt.

Die Virustiter der Vergleichsprobe, des Eluates und der Waschlösung wurden analog Beispiel 10 bestimmt. Der Virustiter (logKID₅₀/ml) der verdünnten Ausgangssuspension betrug 4,4. Im Eluat und der Waschlösung war keine Infektiosität mehr nachweisbar.

### Beispiel 13 Virusabreicherung aus HCV-positivem Plasma

300 mg Polymerpartikel (hergestellt analog Herstellungsbeispiel 2) mit einem mittleren Porenradius von 427nm, einer Porosität von 0,43 sowie einer spezifischen Oberfläche von 88 m²/g wurden analog Beispiel 11 in Säulen gefüllt. Die Partikel wurden mit PBS konditioniert.

5 ml einer Plasmapräparation von HCV-positivem Plasma wurden mit Hilfe einer Spritzenpumpe mit einem konstanten Fluß von 0,25 ml/min durch die Partikel-gefüllte Säule gepumpt. Das Eluat (5 ml) wurde aufgefangen und bis zur Bestimmung des Virustiters bei -80°C eingefroren. Als Vergleichsprobe wurde eine 1:2 (entsprechend der Verdünnung der Plasmapräparation nach Elution von der Säule) mit PBS verdünnte Plasmapräparation mitgeführt.

Die Virustiter des Eluates und der Vergleichsprobe wurden analog Beispiel 10 bestimmt. Der Virustiter der Vergleichsprobe betrug 25.910 IU/ml, der Virustiter des Eluates lag unterhalb der quantitativen Nachweisgrenze von 615 IU/ml. Das entspricht einer Virusabreicherung von >97%.

### Beispiel 14 Abreicherung von BVDV aus Blut

300 mg Polymerpartikel (hergestellt analog Herstellungsbeispiel 3) mit einem mittleren Porenradius von 131nm, einer Porosität von 0,51 sowie einer spezifischen Oberfläche von 105 m²/g wurden in PBS suspendiert und analog Beispiel 11 in eine Säule gefüllt. Vor Versuchsbeginn wurden die Partikel mit Dextran 40 Infusionslösung konditioniert.

5 ml einer Blutpräparation aus humanem Hirudinblut, der eine hochtitrige BVDV-Suspension zugesetzt wurde, wurden mit Hilfe einer Spritzenpumpe mit einem konstanten Fluß von 0,1 ml/min durch die Partikel-gefüllte Säule gepumpt. Das Eluat (5 ml) wurde zur Abtrennung der Blutzellen 10 min bei 2200 g zentrifugiert und bis zur Bestimmung des Virustiters bei -80°C eingefroren. Als Vergleichsprobe wurde die 1:2 (entsprechend der Verdünnung der Blutpräparation nach Elution von der Säule) mit Dextran 40 Infusionslösung verdünnte Blutpräparation mitgeführt.

Die Virustiter der aus dem Eluat und der Vergleichsprobe gewonnnenen Überstände wurden analog Beispiel 10 bestimmt. Der Virustiter der Vergleichsprobe, angegeben in logKID₅₀/ml, wurde mit 3,9 bestimmt, der Virustiter des Eluates mit 2,4. Das entspricht einer Virusabreicherung von 97%.

## Patentansprüche

1. Adsorptionssystem zur Entfernung von Viren und viralen Bestandteilen aus physiologischen Flüssigkeiten, wobei das Adsorptionssystem als Adsorbermaterial Partikel mit einem durchschnittlichen Durchmesser von 20 bis 500 µm umfasst, die eine Polymermatrix mit Durchgangsporen aufweisen.

2. Adsorptionssystem nach Anspruch 1, wobei der Porenradius der Durchgangsporen zwischen 25 und 1000 nm beträgt.

3. Adsorptionssystem nach Anspruch 1 oder 2, wobei die Porosität der Partikel zwischen 0,25 und 0,75 liegt.

4. Adsorptionssystem nach einem der Ansprüche 1 bis 3, wobei die Partikel eine Polymermatrix aufweisen, die von Methacrylsäure als Monomeren abgeleitete Einheiten umfasst.

5. Adsorptionssystem nach Anspruch 4, wobei die Polymermatrix aus einem Methacrylsäureester-enthaltenden Copolymer mit einem Vernetzungsmittel gebildet ist.

6. Adsorptionssystem nach einem der Ansprüche 1 bis 5, wobei auf die Oberfläche der Partikel eine die Hämokompatibilität verbessernde Substanz aufgebracht ist.

7. Adsorptionssystem nach Anspruch 6, wobei die Partikel eine Polymermatrix aufweisen, die von Methacrylsäure abgeleitete Einheiten beinhaltet und die die Hämokompatibilität verbessernde Substanz an eine Polyethylenglykolkette gebunden ist.

8. Adsorptionssystem nach einem der Ansprüche 6 oder 7, wobei die die Hämokompatibilität verbessernde Substanz ein Polyorganosiloxan ist.

9. Verfahren zur Entfernung von Viren oder Virenbestandteilen aus Blut oder Plasma, umfassend das in Kontakt bringen des Blutes oder Blutplasmas mit einem Adsorptionssystem nach einem der Ansprüche 1 bis 8.

10. Adsorbermaterial, umfassend Partikel mit einem durchschnittlichen Durchmesser von 20 bis 500 µm, die eine Polymermatrix mit Durchgangsporen aufweisen, zur Verwendung in einem Adsorptionssystem nach einem der Ansprüche 1 bis 8.

11. Verwendung eines Adsorptionssystems nach Anspruch 1 bis 9 oder eines Adsorbermaterials nach Anspruch 10 zur Behandlung einer Viruserkrankung.

12. Verwendung nach Anspruch 11, wobei es sich bei der Viruserkrankung um eine Erkrankung, ausgewählt aus Hepatitis C, Hepatitis B, AIDS oder viralen haemorrhagischen Fiebern handelt.
